# EUROPEAN PATENT SPECIFICATION

(11) **EP 2 762 087 B1**
(45) Date of publication and mention of the grant of the patent: **30.08.2017**
(21) Application number: 12834753.1
(22) Date of filing: 25.09.2012
(51) Int. Cl.: A61B 17/06

(54) **SUTURE NEEDLE**
NÄHNADEL
AIGUILLE À SUTURE

(30) Priority: 26.09.2011 JP 2011208646
(43) Date of publication of application: 06.08.2014
(73) Proprietor: MANI, INC., Utsunomiya-shi, Tochigi 321-3231 (JP)
(72) Inventor: AKUTSU, Shinichi, Utsunomiya-shi, Tochigi 321-3231 (JP); ISHIDA, Takashi, Utsunomiya-shi, Tochigi 321-3231 (JP)
(74) Representative: Isarpatent
(86) International application number: PCT/JP2012/074477
(87) International publication number: WO 2013/047472

(56) References cited:
- EP-A1- 1 080 693
- EP-A1- 2 226 019
- JP-A- H04 307 051
- JP-A- 2002 224 485
- JP-A- 2010 029 570
- RU-C1- 2 020 883
- US-A- 4 182 341
- US-A1- 2005 149 067

## Description

### Technical Field

The present invention relates to a suture needle used in tendon suturing, ophthalmic operations, etc. It particularly relates to a suture needle to which both ends of a single suture thread or two suture threads are attached to a base end surface, and a manufacturing method thereof.

### Background Art

A suture needle with a suture thread (eyeless suture needle) having a blind hole open in the base end surface of the suture needle along the axis thereof and an end of the suture thread inserted in the hole and crimped has been used conventionally. Of such suture needles, as an ophthalmic needle or a surgical needle with a looped thread used in tendon suturing, for example, there is a needle having both ends of a single suture thread attached to a blind hole where the suture thread forms a loop (See Patent Document 1) and a needle where two suture needles are tied together by two suture threads.

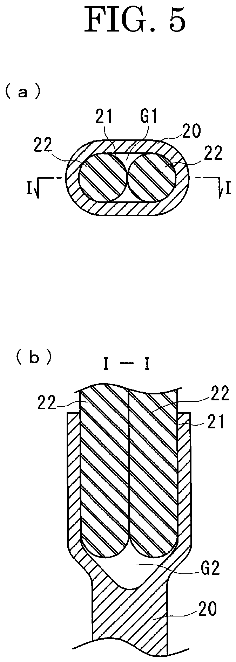

FIG. 5 is a diagram illustrating a base end of a conventional surgical needle with a looped thread described in Patent Document 1; where 5(a) is a cross-sectional view of the base end and 5(b) is a cross-sectional view cut along the line I-I of 5(a). Attaching two suture threads 22 in this manner is carried out in the following sequence: a blind hole 21 is opened in a base end surface such that it is large enough to easily accommodate the two suture threads 22, and the blind hole 21 is slightly compressed so that the shape thereof is an ellipse. Afterwards, the two suture threads 22 are inserted in the blind hole 21 that is made into an elliptic shape and the base end is then crimped.

### Prior Art Documents

### Patent Documents

Patent Document 1: JP H04-307052 A

JP 2002 224485 A relates to a suture needle. The suture needle described by this document has a blind opening along the axis from a base end surface of the needle, to allow both ends of a single suture thread or two suture threads to be attached to the needle. The blind hole is crimped to achieve a substantially eight shape, as the cross-sectional shape of the insertion location of the suture strands. EP 1 080 693 A1 relates a haemostatic and/or reinforcing surgical suture system for sternal cerclages. This document discloses the possibility of having a plurality of holes in a base end of a suture needle, for attaching several suture threads.

### DISCLOSURE OF INVENTION

### Problem to be Solved by the Invention

However, in this case, the radius of the blind hole needs to be large enough such that the two suture threads can be inserted easily. Therefore, there is a problem that a thick suture thread cannot be used with this suture needle radius. Moreover, if the blind hole radius is increased, wall thickness of the blind hole becomes thin and cracking or deformation of the blind hole wall when crimping occurs.

Furthermore, the case of inserting two suture threads in an elliptic blind hole has problems that tensile strength of the thread does not increase since a space G1 is easily left between the two suture threads and the blind hole wall when crimping, and a space G2 is formed near the hole bottom and deep insertion of the suture thread to the hole bottom is impossible since radius of the blind hole compressed into an elliptic shape becomes smaller toward the hole bottom.

There is also a problem of complicated work since once a blind hole is formed, at least a total of two dice, one for transforming the hole into an elliptic shape and one for inserting two suture threads and crimping them, are required.

In light of these conditions, the present invention aims to provide a suture needle having a base end surface to which either both ends of a single suture thread or two suture threads are attached; allowing easy and secure attachment even of a thicker suture thread than in the past. Solution to the Problem

The above object is achieved by a suture needle as recited in claim 1. Preferred features of the invention are recited in the dependent claims.

The suture needle according to the present invention is provided with a blind hole along the axis from a base end surface of the suture needle, thereby allowing a suture thread to be attached by inserting and crimping the suture thread in the blind hole. The blind hole is opened in two places in the same base end surface, and both ends of a single suture thread or two suture threads may be attached to the needle.

Here, the blind holes opened in two places are connected so as to make overall shape of the blind holes in the base end surface have a figure eight shape.

Furthermore, at least a part of the inner surface of the blind holes may be an electric discharge processing surface.

Note that 'blind hole' according to the present invention indicates each blind hole opened along the axis of the base end surface, 'overall shape of the blind hole' indicates shape of the entirety of the blind holes in two places, and case where the blind holes are connected indicates a shape of connected blind holes.

### Advantageous Effect of the Invention

Since a suture thread is inserted in the blind holes opened in two places, blind holes having a radius coordinated with thickness of the suture thread should be opened. Therefore, even a thicker suture thread than in the past is easily inserted and tensile strength is improved.

Moreover, if a part of the respective blind holes in the base end surface is connected, namely they are either tangent or have a common portion so that the overall shape is a figure eight, center of the respective blind holes is near center of the base end surface. This makes crimping force when crimping be easily transmitted to the entire periphery of the blind holes, and the respective blind holes easily made into a circular shape. As a result, the suture thread having a cross-sectional circular form may be securely held from all directions, wall thickness may be made thick, and tensile strength may be improved.
Furthermore, formation of at least a part of the blind holes through electric discharging and thereby making at least a part of the inner surface of the blind holes a rough electric discharge processing surface allow a structure that easily holds the suture thread.

### BRIEF DESCRIPTION OF DRAWINGS

FIG. 1 is an oblique view of both ends of a suture thread attached to a suture needle according to the present invention;
FIG. 2 is a drawing illustrating an embodiment where two suture threads are attached;
FIG. 3 illustrates end views of the suture needle; where 3(a) shows an example, not part of the invention, with separated holes and 3(b) shows the embodiment of the invention, with part of the holes connected;
FIG. 4 is a diagram illustrating manufacturing steps of the suture needle according to the present invention; where 4(a) is a state where a round bar is processed, 4(b-1) and 4(b-2) are states after it is subjected to press work, 4(c-1) and 4(c-2) are base end surfaces in which blind holes are opened, and 4(d-1) and 4(d-2) are states after it is subjected to bending work;
FIG. 5 is a diagram illustrating a base end of a conventional surgical needle with a looped thread; where 5(a) is a cross-sectional view of the base end and 5(b) is a cross-sectional view cut along the line I-I of 5(a);
FIG. 6 is a graph showing test results of tensile strength of the conventional surgical needle with a looped thread having a single blind hole; and
FIG. 7 is a graph showing test results of tensile strength of the surgical needle with a looped thread having two blind holes, according to the present invention.

### DESCRIPTION OF EMBODIMENTS

An embodiment according to the present invention is described with reference to accompanying drawings forthwith.

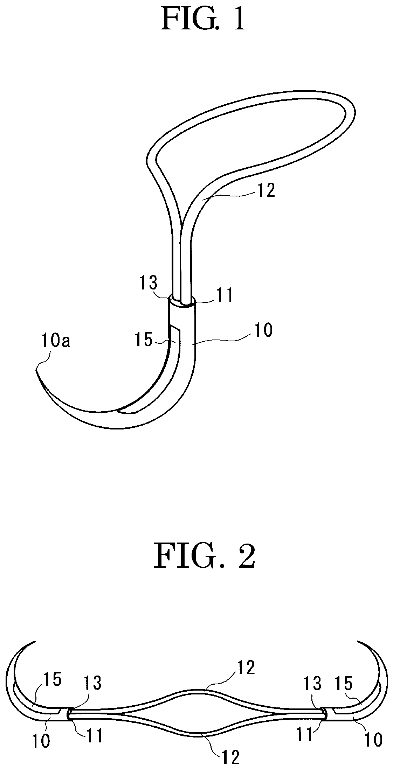

FIG. 1 is an oblique view of both ends of a suture thread attached to a suture needle according to the present invention. FIG. 2 is a drawing illustrating an embodiment where two suture threads are attached.

In the embodiment illustrated in FIG. 1, both ends of a single suture thread 12 are inserted in a blind hole 11 opened in a base end surface 13 of a suture needle 10 and are attached thereto by crimping a base end of the suture needle 10. As a result, the suture thread 12 is attached, forming a loop. Meanwhile, the embodiment of FIG. 2 is a case where two suture needles 10 and 10 are tied together by two suture threads 12 and 12. These suture needles 10 are used for tendon suturing, ophthalmic operations, etc. Two blind holes 11 are opened in the base end surface 13, and while attaching two suture threads 12 is the same, provision of two blind holes 11 in the same base end surface 13 in this manner has not been seen until now. The base end surface 13 of the suture needle 10 according to the present invention is described in detail now.

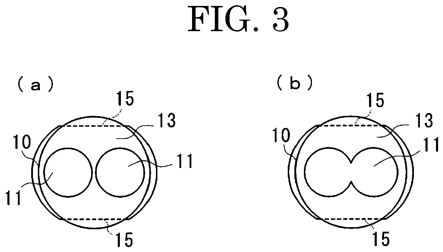

FIG. 3 is an end view of the suture needle where 3(a) illustrates an example of a needle with separated holes and 3(b) illustrates the embodiment of the invention, with part of the holes connected. Both of these cases have two blind holes 11 opened in the same base end surface 13.

Radius of these blind holes 11 is determined in conformity with thickness of the suture threads 12 to be attached. In other words, since the hole radius should be larger than the thread radius, a suture thread of a 0.51 mm radius may be inserted in even a hole radius of 0.52 mm. On the other hand, when inserting two suture threads in a blind hole, a hole radius a little larger than the two suture threads is necessary. Therefore, for a suture needle having a radius of 1.28 mm, only a surgical needle with a looped thread having a radius allowing insertion of two suture threads in a single blind hole having a radius of 0.97 mm can be manufactured. However, in the case of providing the two blind holes 11 as with the present invention, it is possible to attach a suture thread having a radius of 0.53 mm in a loop to a suture needle having a 1.28 mm radius, which has been conventionally impossible. Moreover, since a single blind hole is provided for a single suture thread, the suture thread may be inserted deep until touching the bottom of the blind hole.

The overall shape of the blind holes 11 may be a shape where two blind holes 11 are opened separately in the same base end surface 13 as in FIG. 3(a), or it may be a shape where a figure eight-shaped blind hole 11 results from combining a part of two blind holes 11 as in FIG. 3(b). If the two blind holes are separated or in a figure eight shape, since the space G1 as shown in FIG. 5(a) is not formed when crimping them after the suture thread 12 is inserted, it is easier for the suture thread 12 and wall of the blind holes 11 to adhere to each other than before, thereby improving tensile strength. When the center space between the two blind holes 11 is narrow and the overall shape of the combined blind holes 11 has an elliptic or a long hole shape, since the same space G1 is formed as in the conventional example of FIG. 5(a), tensile strength may not be improved. Therefore, the overall shape of the blind holes 11 needs to be either two separate holes or combined holes in a figure eight shape.

Upon thorough research of the overall shape of the two blind holes 11 through further testing by the inventors, it is understood that the case where the two blind holes 11 are opened separately results in having the center of the respective blind holes 11 at a distance from the center of the base end surface 13. This makes crimping force when crimping difficult to be transmitted to the entire periphery of the respective blind holes 11, and the respective blind holes 11 easily made into an elliptic shape, etc. On the other hand, the case where the two blind holes 11 are connected into a figure eight shape results in having the center of the respective blind holes 11 be near to the center of the base end surface 13. This makes crimping force when crimping easily transmitted to the periphery of the respective blind holes 11, and the respective blind holes 11 easily made into a circular form. As a result, there is an advantage that when the overall shape of the blind holes 11 is made into a figure eight, the suture thread 12 having a cross-sectional circular form can be securely held from all directions.

Note that when the suture needle 10 is a curved suture needle in the case where the blind hole 11 is opened in two places, arrangement of the two blind holes 11 is not limited. In other words, depending on operative method, they may be aligned perpendicular to, horizontally to, diagonally crossing etc. the curve (a virtual plane passing through the curve) when viewed from the base end surface 13 of the suture needle.

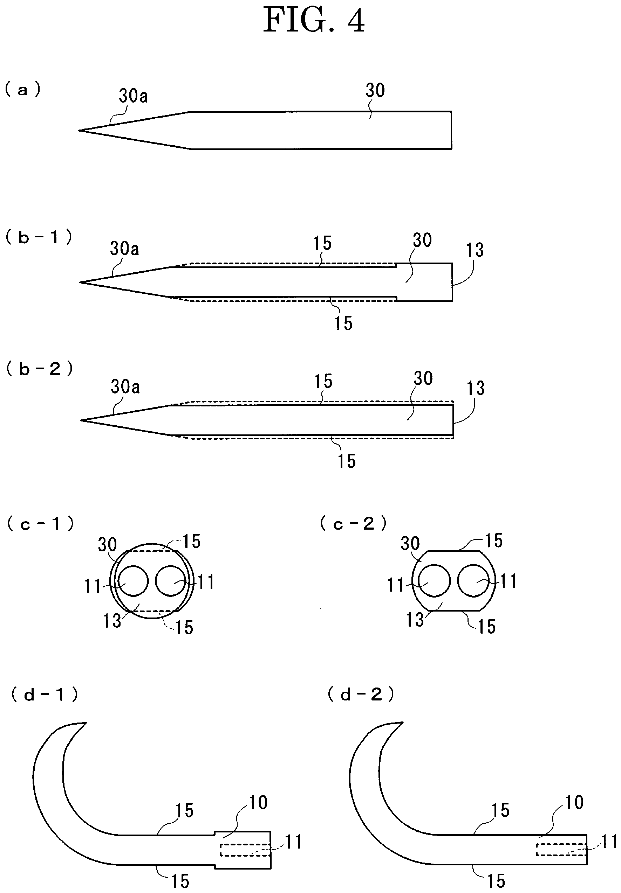

A manufacturing method of such a suture needle is described next. FIG. 4 is a diagram illustrating manufacturing steps of the suture needle according to the present invention; where 4(a) is a state where a round bar is processed, 4(b-1) and 4(b-2) are states after it is subjected to pressing work, 4(c-1) and 4(c-2) are base end surfaces in which blind holes are opened, and 4(d-1) and 4(d-2) are states after it is subjected to bending work. Note that while a curved suture needle having a round cross section is exemplified here, basically the same steps are carried out even for a cutting needle, etc.

In manufacturing a suture needle, a round bar is first processed. A stainless steel wire is wire drawn, obtaining a suture needle 30 partially manufactured up through processing of a point 30a as shown in FIG. 4(a).

Press working is then carried out for providing a flat part 15 on either side of the suture needle 30 as shown in FIGS. 4(b-1) and 4(b-2) so as to securely grasp the suture needle when clamping using a needle holder during surgery. When suturing an affected area using the needle holder, the flat part 15 should be provided on the inner side and the outer side of the curve in the case of the curved suture needle. The flat parts 15 may be formed by press working parts to be clamped by the needle holder, namely, only the main body portion (dotted line portions) of the suture needle 30 excluding the base end and the point 30a as in FIG. 4(b-1), or they may be formed by press working from the main body portion to the base end as shown in FIG. 4(b-2). When the flat part 15 is formed until the base end as in FIG. 4(b-2), the base end may have an elliptical cross section and the blind hole may be formed larger than with the circular cross section of the case of FIG. 4(b-1) etc.

Moreover, this press working also has a meaning of marking in the case of the present invention. This is because due to the two blind holes 11, which are opened in a subsequent step, opened so as to be aligned perpendicular to, horizontally to, diagonally crossing etc. the curve when viewed from the base end surface 13 of the suture needle, the flat parts 15 and 15 formed through press working are markings for confirming orientation of the suture needle 30 when opening the blind holes 11.

FIGS. 4(c-1) and 4(c-2) illustrate the base end surface 13 of the suture needles 30 of FIGS. 4(b-1) and 4(b-2), respectively, in which holes are opened. Since the flat parts 15 and 15 formed through press working are provided on the inner side and the outer side of the curve, the two blind holes 11 and 11 may be opened so as to be aligned in parallel to the surface of the flat part 15 in order to open the holes aligned perpendicular to the curve when viewed from the base end surface 13 as in FIGS. 4(c-1) and 4(c-2). The case of opening holes so as to be aligned horizontally to or diagonally crossing the curve may also confirm orientation by making the surface of the flat parts 15 as a standard.

The hole opening process is carried out for each hole through electric discharging or drilling. However, hole size to be opened by drilling is limited, which makes it difficult to open holes in two places in the base end surface for a small suture needle such as an ophthalmic needle or the like. Moreover, there are problems with opening a hole using a laser beam in that it is difficult to align cores since center of the blind hole and center of the suture needle are different, and sputter splashes into the blind hole as well, making it difficult to insert a suture thread. Meanwhile, these problems do not occur with electric discharging, and a straight hole may be opened even in a narrow place. Note that if at least one of holes in two places is opened through electric discharging, such as opening a first hole through drilling and opening a second hole through electric discharging, it is possible to open holes in two places in the base end surface, including small holes.

In the case of opening the blind hole 11 through electric discharging, the inner surface of the blind hole 11 is a rough electric discharge processing surface. When the inner surface of the blind hole 11 is rough, it is easier to hold a suture thread, thereby improving tensile strength.

Bending of the suture needle 10 is performed last, as shown in FIGS. 4(d-1) and 4(d-2). FIG. 4(d-1) is a drawing of the suture needle 10 of FIG. 4(b-1) after it is subjected to bending work, and FIG. 4(d-2) is a drawing of the suture needle 10 of FIG. 4(b-2) after it is subjected to bending work. Bending direction should be such that the flat parts 15 provided by pressing are on the inner side and the outer side of the curve, as described before. Note that when manufacturing a straight suture needle, the step of bending is unnecessary.

Inserting both ends of a suture thread or two suture threads in the blind hole 11 of the suture needle 10 manufactured in this manner and then crimping it makes it available as a surgical needle with a looped thread used for tendon suturing or an ophthalmic needle. Note that if the suture needle 10 of the present invention is used, crimping of the base end is only required once, and thereby there is an advantage of only needing one die for crimping.

Also note that there are cases where the aforementioned press working illustrated in FIGS. 4(b-1) and 4(b-2) is not carried out depending on type of needle, and cases where it is carried out after the hole opening process. Moreover, the needle may be set up in a device capable of simultaneously performing the press working of FIGS. 4(b-1) and 4(b-2) and the bending of FIGS. 4(d-1) and 4(d-2) so as to simultaneously form the flat parts 15 and the curve.

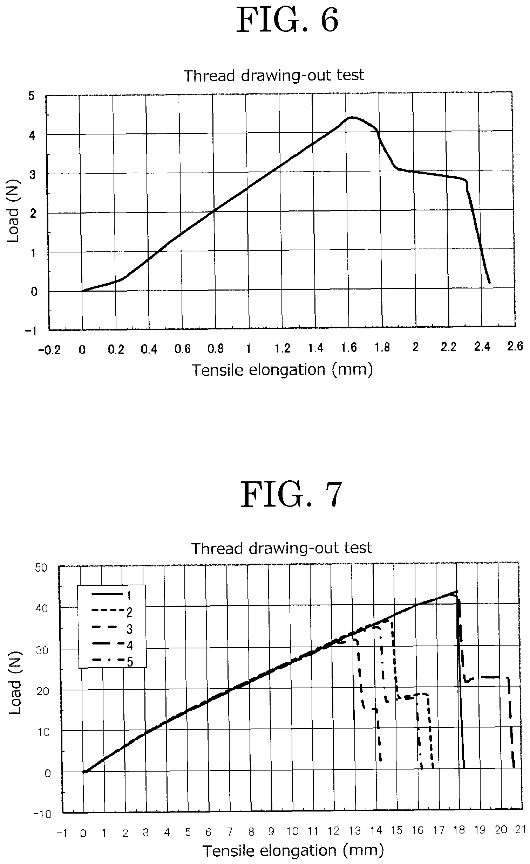

FIG. 6 is a graph showing test results of tensile strength of the conventional surgical needle with a looped thread having a single blind hole. FIG. 7 is a graph showing test results of tensile strength of the surgical needle with a looped thread having two blind holes, according to the present invention.

In both cases, a thread drawing-out test in accordance with USP standards using a load cell is conducted, where both ends of a suture thread having an outer radius of 0.516 mm (USP standard 2) is inserted in a suture needle having an outer radius of 1.28 mm and then crimped using a crimping die compressed to 1.14 mm. The case of the conventional surgical needle with a looped thread having a single blind hole is set to a hole radius of 1.0 mm, and the case of the surgical needle with a looped thread having two blind holes according to the present invention is set to a hole radius of 0.58 mm. Moreover, number of times of crimping is set to ten times for the conventional surgical needle with a looped thread and two times for the surgical needle with a looped thread according to the present invention. Note that the drawing-out standard (USP) for individual values is 6.86N or greater and average value is 17.6N or greater.

According to the test results, the conventional surgical needle with a looped thread has an ultimate load of 4.4N and thereby not fulfilling the standard. The surgical needle with a looped thread according to the present invention, upon testing of five test pieces, has a minimum ultimate load of 31.6N and average value of 37.5N, thereby fulfilling the standards, and has improved tensile strength considerably, eight times or greater than of the conventional surgical needle with a looped thread.

### Explanation of References

10, 20: suture needle
11, 21: blind hole
12, 23: suture thread
13: base end surface
15: flat part

## Claims

1. A suture needle (10) provided with two blind holes (11) along the axis from a base end surface (13) of the suture needle (10), thereby allowing a suture thread to be attached by inserting and crimping the suture thread in the blind holes (11); **characterized in that** each of the blind holes (11) is opened in one place in the same base end surface (13) and the two blind holes are connected so as to make overall shape of the blind holes (11) in the base end surface (13) have a figure eight shape, such that both ends of a single suture thread or two suture threads can be attached to the suture needle (10).

2. The suture needle (10) of Claim 1, wherein the figure eight shaped blind holes (11) results from combining a part of two blind holes (11).

3. The suture needle (10) of either Claim 1 or Claim 2, wherein at least a part of the inner surface of the blind holes (11) is an electric discharge processing surface.

## Patentansprüche

1. Nähnadel (10), die mit zwei Blindlöchern (11) entlang der Achse von einer Endoberfläche der Basis (13) der Nähnadel (10) versehen ist, wodurch ermöglicht wird, dass ein chirurgischer Faden angebracht wird, indem er eingeführt wird und der chirurgische Faden in den Blindlöchern (11) gefaltet wird; **dadurch gekennzeichnet, dass** jedes der Blindlöcher an einer Stelle in derselben Endoberfläche der Basis (13) geöffnet ist und die zwei Blindlöcher derart verbunden sind, dass die Gesamtform der Blindlöcher (11) in der Endoberfläche der Basis (13) die Form der Zahl Acht aufweist, derart, dass beide Enden eines einzelnen Nähfadens oder von zwei Nähfäden an der Nähnadel (10) befestigt werden kann bzw. können.

2. Nähnadel (10) nach Anspruch 1, wobei die Blindlöcher (11) in der Form der Zahl Acht durch die Kombination eines Teils von zwei Blindlöchern (11) entstehen.

3. Nähnadel (10) nach Anspruch 1 oder Anspruch 2, wobei mindestens ein Teil der inneren Oberfläche der Blindlöcher (11) eine Elektroerosionsbearbeitungsoberfläche ist.

## Revendications

1. Aiguille à suture (10) pourvue de deux trous borgnes (11) le long de l'axe à partir d'une surface d'extrémité de base (13) de l'aiguille à suture (10), de manière à permettre à un fil de suture d'être attaché par insertion et sertissage du fil de suture dans les trous borgnes (11) ; **caractérisée en ce que** :
chacun des trous borgnes (11) est ouvert à un emplacement de la même surface d'extrémité de base (13) et les deux trous bornes sont reliés, de manière donner à la forme générale des trous borgnes (11) sur la surface d'extrémité de base (13) une forme de chiffre huit, de sorte que les deux extrémités d'un seul fil de suture ou de deux fils de suture peuvent être attachées à l'aiguille à suture (10).

2. Aiguille à suture (10) selon la revendication 1, dans laquelle les trous borgnes en forme de chiffre huit (11) résultent de la combinaison d'une partie de deux trous borgnes (11).

3. Aiguille à suture (10) selon l'une des revendications 1 et 2, dans laquelle au moins une partie de la surface intérieure des trous borgnes (11) est une surface de traitement par décharge électrique.
